# EUROPEAN PATENT SPECIFICATION

(11) **EP 0 725 751 B1**
(45) Date of publication and mention of the grant of the patent: **28.04.1999**
(21) Application number: 93925046.0
(22) Date of filing: 26.10.1993
(51) Int. Cl.: B65D 65/38, G01N 27/12, G01R 27/22, A61N 1/04

(54) **MEDICAL ELECTRODE PACKAGE**
VERPACKUNG FÜR EINE MEDIZINISCHE ELEKTRODE
EMBALLAGE POUR ELECTRODE MEDICALE

(43) Date of publication of application: 14.08.1996
(73) Proprietor: Surviva-link Corporation, Minneapolis, MN 55343 (US)
(72) Inventor: GILMAN, Byron L., Plymouth, MN 55447 (US); KROLL, Karl J. F., Maple Grove, MN 55369 (US)
(74) Representative: Hale, Peter
(86) International application number: US9310234
(87) International publication number: WO9511836

(56) References cited:
- US-A- 3 198 329
- US-A- 3 265 945
- US-A- 4 029 086
- US-A- 4 034 854

## Description

This invention relates to electro-medical apparatus and methods and particularly to packaging structures for medical electrode apparatus. The packaging structures are particularly useful for housing medical electrodes prior to use. And, the packaging structures have features that will allow the stored medical electrodes to be periodically tested during storage.

In the past, various electrode devices and/or methods, including packaging schemes have been used and proposed. However; these devices are generally complex, inefficient to use and have significant shortcomings. Specifically, most electrodes and packaging structures therefore, do not provide the ability to test for readiness after, typically, long periods of storage without compromising the sterility and performance of the electrode. For example, US 4,034,854 discloses an electrode assembly having an electrode with a base layer and a gel layer overlaying the base layer. Enclosing the electrode is an envelope, typically made of plastic or metal foil. However, in order to test the electrode, the envelope has to be removed, thereby compromising the sterility and performance of the electrode.

Despite the need for an electrode device, and packaging therefor, in the art, which overcomes the limitations and problems of the prior art, none insofar as is known has been proposed or developed. Accordingly, it is an object of the present invention to provide an electrode system which is relatively simple to manufacture and to use, which is effective at delivering high currents and voltages for use in cardiac defibrillation, which is stable and has a long shelf life, and which permits periodic testing of electrode viability and/or functionality without degrading electrode quality.

According to the present invention, there is provided a electrode as specified in claim 1. Some preferred features of the invention are specified in the dependent claims.

The benefits of this invention will become clear from the following description by reference to the drawings.

### BRIEF DESCRIPTION OF THE DRAWINGS

FIG. 1 is a top view of one embodiment of the medical electrode system of the present invention;
FIG. 2 is a side view, partially in crossestion, of the electrode of FIG. 1, and further showing a test apparatus therefor;
FIG. 3 is a top view of another embodiment of the electrode;
FIG. 4 is a side view of the electrode shown in FIG. 3;
FIG. 5 is atop view of another embodiment of the electrode;
FIG. 6 is a side view of the electrode shown in FIG. 5;
FIG. 7 is a top view of another embodiment of the electrode;
FIG. 8 is a side view of the electrode shown in FIG. 7;
FIG. 9 is a top view of another embodiment of the electrode;
FIG. 10 is a side view of the electrode shown in FIG. 9;
FIG. 11 is a top view of another embodiment of the electrode;
FIG. 12 is aside view of the electrode shown in FIG. 11;
FIG. 13 is a top view of another embodiment of the electrode;
FIG. 14 is a side view of the electrode shown in FIG. 13;
FIG. 15 is a detailed view of a snap-type connection.
FIG. 16 is a detailed view of a top member of the snap connection shown in FIG. 15;
FIG. 17 is a crossectional view of a resistive layer; and
FIG. 18 is a crossectional view of packaging material layers.

### DESCRIPTION OF THE PREFERRED EMBODIMENT

The present invention provides an electrode system for use in the medical arts, particularly in cardiac defibrillation. The system includes cooperating electrode embodiments, packaging embodiments and test instrumentation. The system provides convenient, secure and sterile storage means for electrodes which are easy to manufacture and use. The system so provides a means of periodically testing the operability of the stored electrodes without destroying the packaging or electrodes, and without compromising the sterility of the materials. The teachings of this invention are applicable to electrodes used for either or both transmitting or receiving, although they are particularly beneficial for use in electrodes used in cardiac defibrillation, which electrodes require transfer of particularly high currents and voltages and a high confidence lead in readiness.

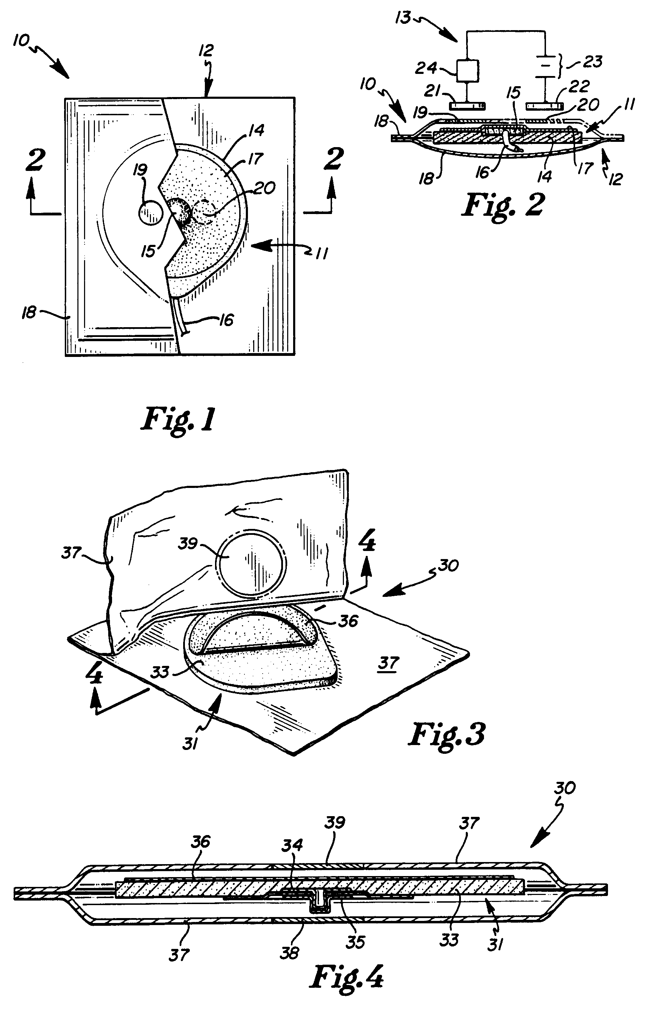

Referring to FIGS. 1 and 2 an embodiment of the packaged electrode system 10 is shown to comprise an electrode 11 and a package or enclosure 12. Also shown in FIG. 2 is a test apparatus 13. The electrode 11 is shown to comprise a non-conductive base or backing layer 14, a conductor or conductive layer 15, a lead 16, and a conductive contact layer 17. The base layer 14 is preferably constructed of a thin, flexible polymeric substance such as a urethane foam, or a polyester or polyolefin laminate which provides structural base and insulative properties. Although the base layer 14 is shown to have a surface area which is substantially coextensive with the surface of the contact layer 17, it alternatively may be slightly larger. In such larger configurations, the base layer 14 may have a pressure sensitive adhesive disposed on its patient contact side for increased adhesion to the patient body.

The conductive layer 15 is shown to be disposed on the first or patient side of base layer 14. It functions to transfer (disperse) current or voltage from the lead 16 (or to the lead in a sensing application) to the patient contact layer 17. Although the conductive layer 15 is shown to have a surface area which is smaller than that of the base layer 14 or contact layer 17, it may alternatively have a dimension which is larger than that shown, or even one which is coextensive with the base and contact layers 14 and 17. The conductive layer 15 is preferably a homogeneous, solid, thinly deposited metallic substance, or a conductive ink material. Alternatively, the conductive layer 15 may be formed of a flexible mesh material, a conductive adhesive or a deposited ink pattern. Flexible conductive ink compounds known in the art have a conductive filler of Gold, Silver, Aluminum or other conductive materials.

The lead 16 is preferably an insulated wire conductor which extends from a mating point with the conductive layer 15, through the base layer 14, and then has a freely movable end. Various alternatives of this lead 16 design exist and are useable consistent with the general teachings of the invention, including but not limited to uninsulated wire conductors and conductive strips or traces deposited between the contact layer 17 and the base 14 or conductive layers 15. Such a trace or strip may also extend just beyond the base layer 14 for connection with an ancillary connection means such as a wiring harness including conductive clip means.

The conductive contact layer 17 is preferably a thin layer of semi-liquid gel material. The gel maintains direct electrical contact with the skin, to reduce variations in conductance, and it permits such contact for long periods of time. The gel is a conductive, gelatinous compound which is also flexible for contoured adhesion to the body of a patient. The gel also preferably has a pressure sensitive, moisture resistant adhesive property. Compounds having these characteristics have been developed by Minnesota Mining and Manufacturing, Medtronic, and Lec Tec (Synkara TM), Corporations, all of Minnesota, U.S.A. Generally, these compounds have low resistivities. The contact layer 17 is for direct contact with the patient's body to transfer current or voltage thereto or therefrom. Overall, although the electrode 11 and its constituent elements are shown to have circular configurations, they may alternatively be formed in various other shapes such as rectangular or square patches.

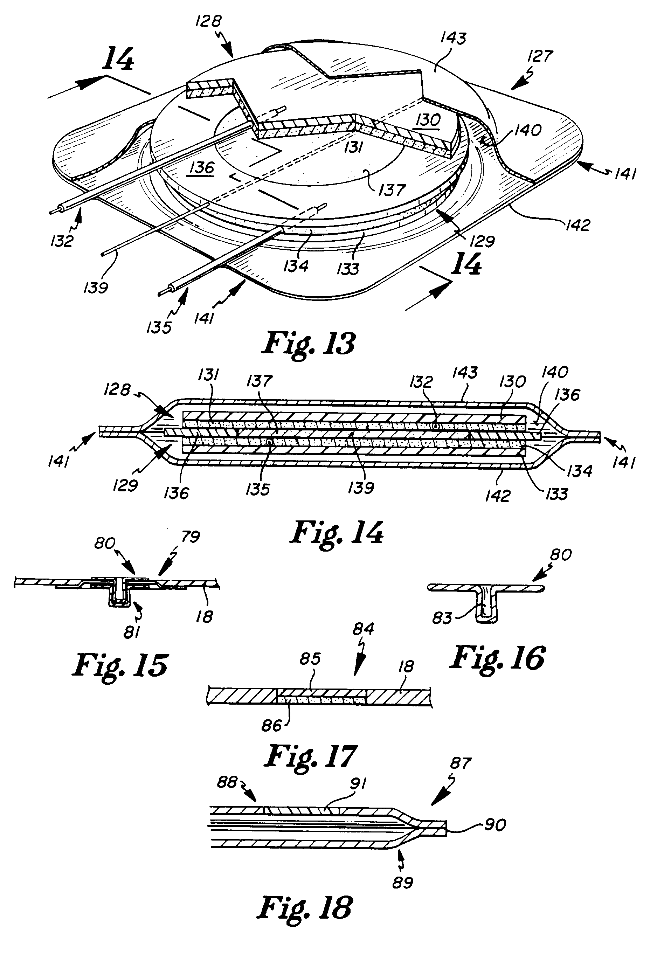

The package structure 12 is shown to have a envelope-like structure formed of a substantially continuous thin, homogeneous layer 18 of a polymeric, preferably non-gas permeable, material. Alternatively, a shown in FIG. 18, the package 87 embodiment may have a pouch-like structure formed of a pair of thin, flat homogeneous layers 88 and 89 which are sealed or otherwise merged together at their peripheries or outer edges 90. And, although the package 12 is shown to have a rectangular configuration various other configurations and shapes are also useable consistent with the invention.

The package further comprises a pair of conductive connectors 19 and 20 which are separated a predetermined distance from one another for contact with separate areas of the contact layer 17 of the enclosed electrode 11. The connectors 19 and 20 are conductive areas which are shown to have a unitary construction with the package layer 18. The contacts 19 and 20 may alternatively be formed of thin layer strips of conductive material, or a printed conductive ink, disposed on the interior side of the package layer 18, extending from contact nodes to peripheral contact areas on the exterior of the package 18. Yet another snap-type embodiment 79 is shown in FIGS. 15 and 16 including a connective member 80 disposed on one side of the base layer 18, and a current dispersion member 81 disposed on the opposite side and being connected to the upper member 80 via an aperture in the base 18. The upper member 80 is shown to have a base 82 and a mating notch 83 for coupling the lower member 81.

Referring to FIG. 2, the system 10 of the present invention also comprises a test apparatus 13. The test apparatus 13 includes a current source 23, preferably a battery, test circuitry 24, preferably including measurement components and status indication components such as an analog meter, LCD digital display or light emitting diodes, and connectors 21 and 22 for coupling with the package 12 connectors 19 and 20. In use, the test apparatus 13 is connected to the package connectors 19 and 20. The test circuitry 24 is then activated to form a closed current loop to determine whether continuity exists with respect to the enclosed electrode 11, thereby indicating whether the electrode 11 is still functional. Additionally, a load 86 formed of for example a conductive and semi-conductive material layers 85 and 86, may be added to the current loop as for example is shown in FIG. 17, for purposes of measuring the magnitude of current flow for more precise measurement of electrode 11 condition.

In the case of the electrode system embodiment 10, a current loop is formed including the connector 19, the gel of the contact layer 17 (along a substantially horizontal plane), and the connector 20 which is located at a remote location on the contact layer 14 with respect to the connector 19. Current conducts easily in fresh, semi-liquid gel of the contact layer 17. In contrast, no current conducts, or current conduction is attenuated, in stale, dried gel. This is indicative of the need to dispose of the stored electrode without using it. And, this condition is determinable without the need to open the package 12 and thereby risk compromising the freshness or sterility of a viable electrode 11.

Referring to FIGS. 3 and 4, another embodiment of the packaged electrode system 30 is shown to comprise an electrode 31 and a package or enclosure 32. The electrode 31 is shown to comprise a non-conductive base layer 33, and a conductive gel layer 36. A conductive snap-type connector having a connection member 35 disposed on one side and a current dispersion member 34 disposed on the second side is also shown. The package 32 is shown to have at least one body layer 37 with a pair of contacts 38 and 39 disposed at predetermined locations to electrically connect with the gel layer 36 and contact 35. In a test mode, a current loop is formed between the connector 39, gel layer 36, connector portions 34 an 35 and connector 38.

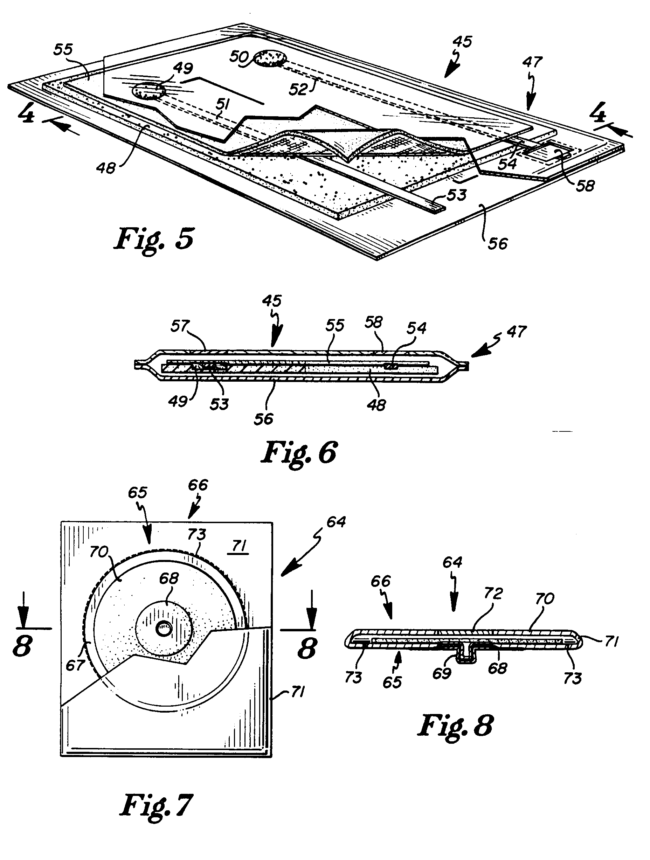

Referring to FIGS. 5 and 6, another embodiment of the packaged electrode system 45 is shown to comprise an electrode 46 and an enclosure 47. The electrode 46 is shown to comprise a non-conductive base layer 48, a conductive gel layer 55, and a pair of separate conductive layers 49 and 50, each of which are shown to have a lead 51 and 52 extending therefrom and terminating in a connective node 53 and 54. The lead pair 51 and 52 (and layer pair 49 and 50) provide a redundant circuit path for increased reliability of use in emergency settings. The package 47 is shown to have at least one body layer 56 with a pair of contacts 57 and 58 disposed at predetermined locations to electrically couple with connective nodes 53 and 54. In a test mode, a current loop is formed between a connector 57 or 58, it's respective connective node 53 or 54 and lead 51 or 52, and its respective conductive layer 49 or 50. In a properly functioning electrode 46, current conducts through the gel 55 from one conductive layer 49 to the other 50, and then back to the test apparatus through the above-mentioned path.

Referring to FIGS. 7 and 8, another embodiment of the packaged electrode system 64 is shown to comprise an electrode 65 and a unitary package 66. The electrode 65 is shown to comprise a non-conductive base layer 67, and a conductive gel layer 70. A snap-type connector with members 68 and 69 electrically couples the gel layer 70.

The package 66 is shown to have at least one body layer 71 which is coupled to the electrode 65 base layer 67 at tear-away perforated lines 73. A connector 72 is shown disposed for contact with the electrode 65 gel layer 70. In a test mode, a current loop is formed between the connector 72, the gel layer 70, and the connector members 68 and 69.

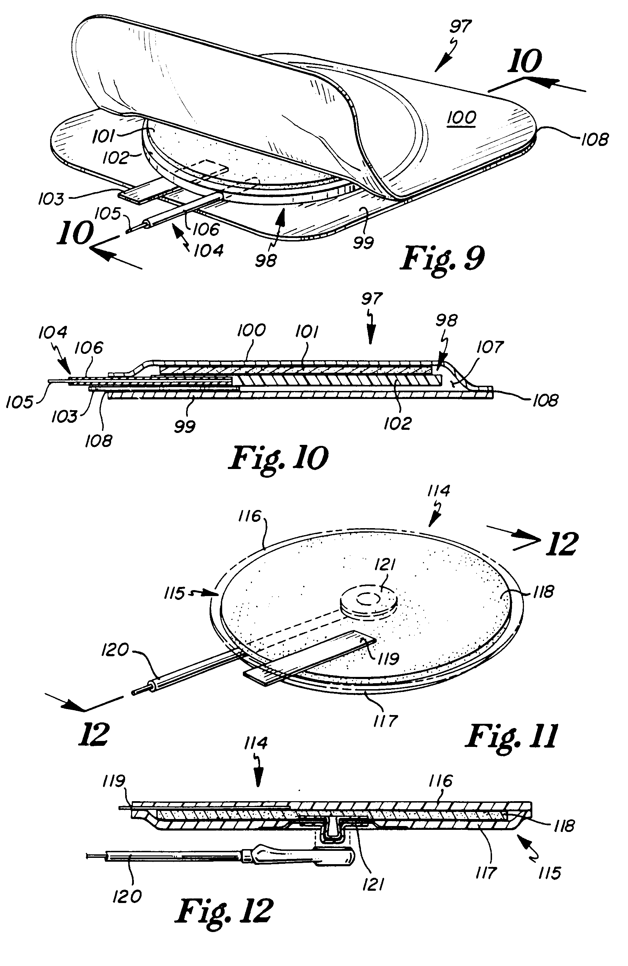

Referring to FIGS. 9 and 10, another embodiment of the packaged electrode system 97 is shown to comprise an electrode 98 and a package. The electrode 98 is shown to comprise a non-conductive base layer 101, a conductive gel layer 102, and a lead 104 having a conductor 105 and an insulator 106, which is shown to be embedded directly in the gel layer 102. Alternatively, the lead may be connected to a conductive current dispersion layer (not shown). A conductive test strip 103 is also shown to be adhered to the surface of the gel 102 at a location remote from the lead 104 for test purposes, and which is designed to release from the gel 102 upon removal of the package layer 99.

The package is shown to have a pair of layers 99 and 100 which overlap to form an interior cavity 107 and are sealingly connected at their peripheries 108. In a test mode, a current loop is formed between the lead 104, the gel layer 102 and the test strip 103, which like the lead 104 is shown extended through the package periphery 108 for contact with an external test apparatus.

Referring to FIGS. 11 and 12, another embodiment of the packaged electrode system 114 is shown to comprise an electrode 115 and an enclosure. The electrode 115 is shown to comprise a non-conductive base layer 117, and a conductive adhesive gel layer 118 which is connected to a snap-type connection node 121 or the like, and an associated lead 120. The package is shown to comprise a single top layer of non-conductive material 116 which is laminated or adhesively mated to the electrode base layer 117. In use the gel layer 118 is removable from the package layer 116. A test strip 119 is disposed on the interior of the package, adhesively connected to the gel layer 118, and extending to the package exterior. In a test mode, a current loop is formed between the lead 120, node 121, gel layer 118 and the test strip 119.

Referring to FIGS. 13 and 14, another embodiment of the packaged electrode system 127 is shown to comprise a pair of electrodes 128 ad 129 and a package. The electrodes 128 and 129 are shown to comprise non-conductive base layers 130 and 133, and conductive gel layers 131 and 134. Leads 132 and 135 extend from the respective gel layers 131 and 134. The package is shown to have a pair of overlapping layers 142 and 143 which are sealed at their peripheries 141 to form an enclosure 140 housing the electrodes 128 and 129. Importantly, the electrodes 128 and 129 are oriented with their respective gel layers 131 and 134 mating with 8 resistive layer 137 (and an optional separator layer 136) formed of a conductive/resistive material as known in the art. A conductive lead 139 or strip extends from the resistive layer through the package periphery 141, as do the electrode leads 132 and 135.

In a test mode, a current loop is formed between, for example, a lead 132, a gel layer 131, the resistive layer 137, and the remaining gel layer 134 and lead 135. The circuit can be altered to include the lead 139.

## Claims

1. A packaged disposable defibrillator electrode (10, 127) including a first disposable defibrillator electrode (11, 128) comprising:
a base layer(14, 130); and
a patient-engaging conductive gel layer (17, 131) overlaying the base layer (14, 130); and
a generally gas-impermeable package (12) surrounding the first electrode (11),
characterised in that a first insulated lead wire (16, 132) is provided having first and second ends, the first end mounted to the first electrode (11) and electrically interconnected to the conductive gel layer (17) and the second end configured for electrical interconnection to a defibrillator, and wherein the generally gas-impermeable package (12) also surrounds the first end of the first lead wire (16) to protect the first electrode prior to use, with the first lead wire (16) extending from the package (12) to enable the second end to be interconnected to a defibrillator prior to the opening of the package (12) and use of the first electrode (11).

2. The electrode of claim 1
further including:
a second disposable defibrillator electrode (129) comprising:
a base layer (133); and
a patient-engaging conductive gel layer (134) overlaying the base layer (133);
a second insulated lead wire (135) having first and second ends, the first end mounted to the second electrode (129) and electrically interconnected to the conductive gel layer (134) and the second end configured for electrical interconnection to a defibrillator,
and wherein
the package also surrounds the second electrode (129) and the first end of the second lead wire to protect the second electrode (129) prior to use, with the second lead wire extending from the package to enable the second end to be interconnected to a defibrillator prior to the opening of the package and use of the second electrode (129).

3. The electrode of claim 2 wherein the conductive gel layers (131 and 134) of the first and second electrodes (128 and 129) are electrically interconnected to one another within the package to form an electrical circuit between the second ends of the first and second lead wires (132 and 135) and enable testing of the electrical characteristics of the first and second electrodes (128 and 129) prior to the opening of the package.

4. The electrode of claim 3 wherein the first and second electrodes (128 and 129) are positioned within the package with the conductive gel layers (131 and 134) oriented toward one another; and which further includes a separator layer (136) between the conductive gel layers (131 and 134) of the electrodes to enable the electrical interconnection of the conductive gel layers (131 and 134).

5. The electrode of claim 1
wherein the package (12) includes a pair of generally gas-impermeable layers (88 and 89) having outer edges sealed together, with the lead wire extending between the layers.

6. The electrode of claim 5 wherein the gas-impermeable layers (88 and 89) of the package (12) include polymeric material.

7. The electrode of claim 1 wherein the package (12) includes generally gas-impermeable polymeric material.

8. The electrode of claim 1 wherein the base layer (16, 132) of the first electrode includes a layer of flexible, nonconductive material, and the conductive gel layer (17, 131) includes an adhesive conductive gel layer.

9. The electrode of claim 8 wherein the package (12) includes a pair of generally gas-impermeable layers (88 and 89) having outer edges sealed together, with the lead wire extending between the layers.

10. The electrode of claim 9 wherein the gas-impermeable layers (88 and 89) of the package include polymeric material.

11. The electrode of claim 8 wherein the package (12) includes generally gas-impermeable polymeric material.

## Patentansprüche

1. Verpackte Einweg-Defibrillatorelektrode (10, 127) mit einer ersten Einweg-Defibrillatorelektrode (11, 128), die
eine Grundschicht (14, 130) und
eine der Grundschicht (14, 130) überlagerte, leitende Gelschicht (17, 131) zum Aufbringen auf den Patienten aufweist,
einer die erste Elektrode (11) umgebenden, im wesentlichen gasundurchlässigen Verpackung (12),
dadurch gekennzeichnet, daß ein erster isolierter Leitungsdraht (16, 132) mit einem ersten und einem zweiten Ende vorgesehen ist, dessen erstes Ende an der ersten Elektrode (11) angebracht und mit der leitenden Gelschicht (17) elektrisch verbunden ist, und dessen zweites Ende für eine elektrische Verbindung mit dem Defibrillator ausgestaltet ist, wobei die im wesentlichen gasundurchlässige Verpackung (12) auch das erste Ende des ersten Leitungsdrahtes (16) umgibt, um die erste Elektrode vor dem Gebrauch zu schützen und wobei der erste Leitungsdraht (16) aus der Verpackung (12) hinausragt, so daß das zweite Ende vor dem Öffnen der Verpackung und vor dem Gebrauch der ersten Elektrode (11) mit einem Defibrillator verbunden werden kann.

2. Elektrode nach Anspruch 1
mit
einer zweiten Einweg-Defibrillatorelektrode (129), die
eine Grundschicht (133) enthält und
eine der Grundschicht (133) überlagerte leitende Gelschicht (134) zum Aufbringen auf den Patienten,
einem zweiten isolierten Leitungsdraht (135) mit einem ersten und einem zweiten Ende, dessen erstes Ende an der zweiten Elektrode (129) angebracht und mit der leitenden Gelschicht (134) elektrisch verbunden ist, und dessen zweites Ende für die elektrische Verbindung mit einem Defibrillator ausgestaltet ist, wobei
die Verpackung auch die zweite Elektrode (129) und das erste Ende des zweiten Leitungsdrahtes umgibt, um die zweite Elektrode (129) vor dem Gebrauch zu schützen, wobei der zweite Leitungsdraht aus der Verpackung herausragt, so daß das zweite Ende vor dem Öffnen de Verpackung und vor dem Gebrauch der zweiten Elektrode (129) mit einem Defibrillator verbunden werden kann.

3. Elektrode nach Anspruch 2, bei der die leitenden Gelschichten (131 und 134) der ersten und der zweiten Elektrode (128 und 129) innerhalb der Verpackung miteinander elektrisch verbunden sind und einen elektrischen Stromkreis zwischen den zweiten Enden des ersten und des zweiten Leitungsdrahtes (132 und 135) bilden, wodurch vor dem Öffnen der Verpackung eine Prüfung der elektrischen Eigenschaften der ersten und der zweiten Elektrode (128 und 129) möglich wird.

4. Elektrode nach Anspruch 3, bei der die erste und die zweite Elektrode (128 und 129) innerhalb der Verpackung angeordnet und die leitenden Gelschichten (131 und 134) zueinander ausgerichtet sind, wobei die Elektrode eine Trennschicht (136) zwischen den leitenden Gelschichten (131 und 134) der Elektroden aufweist, um eine elektrische Verbindung der leitenden Gelschichten (131 und 134) herzustellen.

5. Elektrode nach Anspruch 1, bei der die Verpackung (12) ein Paar von im wesentlichen gasundurchlässigen Schichten (88 und 89) enthält, deren äußere Kanten miteinander verschlossen sind, wobei der Leitungsdraht zwischen den Schichten hervorragt.

6. Elektrode nach Anspruch 5, bei der die gasundurchlässigen Schichten (88 und 89) der Verpackung (12) ein Polymermaterial enthalten.

7. Elektrode nach Anspruch 1, bei der die Verpackung (12) im wesentlichen gasundurchlässigenes Polymermaterial enthält.

8. Elektrode nach Anspruch 1, bei der die Grundschicht (16, 132) der ersten Elektrode eine Schicht flexiblen, nicht leitenden Materials enthält, und bei der die leitende Gelschicht (17, 131) eine adhäsive leitende Gelschicht enthält.

9. Elektrode nach Anspruch 8, bei der die Verpackung (12) ein Paar von im wesentlichen gasundurchlässigen Schichten (88 und 89) enthält, deren äußere Kanten miteinander verschlossen sind, wobei der Leitungsdraht zwischen den Schichten hervorragt.

10. Elektrode nach Anspruch 9, bei der die gasundurchlässigen Schichten (88 und 89) der Verpackung Polymermaterial enthalten.

11. Elektrode nach Anspruch 8, bei der die Verpackung (12) im wesentlichen gasundurchlässigenes Polymermaterial enthält.

## Revendications

1. Electrode de défibrillateur jetable emballée (10, 127) comprenant une première électrode de défibrillateur jetable (11, 128), comprenant :
une couche de base (14, 130) ; et
une couche de gel conducteur (17, 131), à mettre en contact avec un patient, recouvrant la couche de base (14, 130) ; et
un emballage (12) globalement imperméable aux gaz entourant la première électrode (11),
caractérisée en ce qu'un premier fil conducteur isolé (16, 132) est prévu, ayant des première et seconde extrémités, la première extrémité étant montée sur la première électrode (11) et étant électriquement reliée à la couche de gel conducteur (17) et la seconde extrémité étant conformée pour une connexion électrique avec un défibrillateur, et dans laquelle l'emballage (12) globalement imperméable aux gaz entoure également la première extrémité du premier fil conducteur (16) pour protéger la première électrode avant utilisation, le premier fil conducteur (16) s'étendant à partir de l'emballage (12) pour permettre à la seconde extrémité d'être reliée à un défibrillateur avant l'ouverture de l'emballage (12) et l'utilisation de la première électrode (11).

2. Electrode selon la revendication 1, comprenant de plus :
une seconde électrode de défibrillateur jetable (129) comprenant :
une couche de base (133) ; et
une couche de gel conducteur (134), à mettre en contact avec un patient, recouvrant la couche de base (133) ;
un second fil conducteur isolé (135) ayant des première et seconde extrémités, la première extrémité étant montée sur la seconde électrode (129) et étant électriquement reliée à la couche de gel conducteur (134) et la seconde extrémité étant conformée pour une connexion électrique avec un défibrillateur,
et dans laquelle
l'emballage entoure également la seconde électrode (129) et la première extrémité du second fil conducteur pour protéger la seconde électrode (129) avant utilisation, le second fil conducteur s'étendant à partir de l'emballage pour permettre à la seconde extrémité d'être reliée à un défibrillateur avant l'ouverture de l'emballage et l'utilisation de la seconde électrode (129).

3. Electrode selon la revendication 2, dans laquelle les couches de gel conducteur (131 et 134) des première et seconde électrodes (128 et 129) sont électriquement reliées l'une à l'autre à l'intérieur de l'emballage pour former un circuit électrique entre les secondes extrémités des premier et second fils conducteurs (132 et 135) et pour permettre le contrôle des caractéristiques électriques des première et seconde électrodes (128 et 129) avant l'ouverture de l'emballage.

4. Electrode selon la revendication 3, dans laquelle les première et seconde électrodes (128 et 129) sont positionnées à l'intérieur de l'emballage, les couches de gel conducteur (131 et 134) étant orientées l'une vers l'autre ; et qui comprend de plus une couche de séparation (136) entre les couches de gel conducteur (131 et 134) des électrodes pour permettre l'interconnexion électrique des couches de gel conducteur (131 et 134).

5. Electrode selon la revendication 1, dans laquelle l'emballage (12) comprend un couple de couches globalement imperméables aux gaz (88 et 89) ayant des bords extérieurs scellés ensemble, le fil conducteur s'étendant entre les couches.

6. Electrode selon la revendication 5, dans laquelle les couches imperméables aux gaz (88 et 89) de l'emballage (12) comprennent une matière polymère.

7. Electrode selon la revendication 1, dans laquelle l'emballage (12) comprend une matière polymère globalement imperméable aux gaz.

8. Electrode selon la revendication 1, dans laquelle la couche de base (16, 132) de la première électrode comprend une couche de matière souple non conductrice, et la couche de gel conducteur (17, 131) comprend une couche adhésive de gel conducteur.

9. Electrode selon la revendication 8, dans laquelle l'emballage (12) comprend un couple de couches globalement imperméables aux gaz (88 et 89) ayant des bords extérieurs scellés ensemble, le fil conducteur s'étendant entre les couches.

10. Electrode selon la revendication 9, dans laquelle les couches imperméables aux gaz (88 et 89) de l'emballage comprennent une matière polymère.

11. Electrode selon la revendication 8, dans laquelle l'emballage (12) comprend une matière polymère globalement imperméable aux gaz.
